(19) Europäisches Patentamt
European Patent Office
Office européen des brevets

(11) **EP 2 039 284 B1**

(12) **EUROPEAN PATENT SPECIFICATION**

(45) Date of publication and mention
of the grant of the patent:
**25.05.2011 Bulletin 2011/21**

(51) Int Cl.:
***A61B 1/005*** *(2006.01)*

(21) Application number: **08016473.4**

(22) Date of filing: **18.09.2008**

(54) **Endoscope**

Endoskop

Endoscope

(84) Designated Contracting States:
**DE FR GB**

(30) Priority: **19.09.2007 JP 2007242550**
**17.12.2007 JP 2007324448**

(43) Date of publication of application:
**25.03.2009 Bulletin 2009/13**

(73) Proprietor: **FUJIFILM Corporation**
**Minato-ku**
**Tokyo (JP)**

(72) Inventor: **Ashida, Tsuyoshi**
**Ashigara-kami-gun**
**Kanagawa (JP)**

(74) Representative: **Klunker . Schmitt-Nilson . Hirsch**
**Patentanwälte**
**Destouchesstrasse 68**
**80796 München (DE)**

(56) References cited:
**JP-A- 2005 028 018     US-A- 5 469 840**

## Description

BACKGROUND OF THE INVENTION

The present invention relates to an endoscope according to the preamble of claim 1, specifically to an endoscope in which the bending portion provided near the distal end of an insertion section can be bent in a safe and easy manner.

As is well known, endoscopes are inserted into living bodies such as the human body for use in diagnosis or treatment of organs, collection of specimens, and other applications.

As is also well known, an endoscope consists basically of an insertion section to be inserted into the human body, a manipulating section by which the insertion section is manipulated and the endoscope is manipulated for air insufflation/water feeding, a connector (light guide (LG) connector) that is connected to an air insufflation source, a suction pump, etc., and a universal cord (supply hose) for connecting the connector to the manipulating section and the insertion section.

Typically provided near the distal end of the insertion section of the endoscope is a bending portion (angle portion), which can be bent in four directions, up or down and to the right or left, by a manipulating means provided in the manipulating section.

The bending portion is generally bent by pulling it with wires. Specifically, using the manipulating means such as rotatable knobs provided on the manipulating section of the endoscope, pulleys that are rotated by rotating the manipulating means, and wires each of which is fixed at an end to the associated pulley and connected at the other end to the bending portion, the wire for the direction in which the bending portion is to be bent is pulled by the manipulating means, whereupon the bending portion near the distal end of the insertion section is bent.

The endoscope has two wires associated with bending up and down, and associated pulleys and manipulating means for up and down directions, as well as two wires associated with bending to the right and left, and associated pulleys and manipulating means for right and left directions; using these manipulating means, so-called remote manipulation is performed to bend the bending portion in four directions, up or down and to the right or left.

An operator of the endoscope manipulates it while holding the manipulating section which is one of the heaviest parts of the endoscope. In addition, the bending operation by pulling the wires requires a substantial manipulative force in the presence of wire friction and other factors and this imposes considerable burden on the operator. What is more, the manipulative force required to bend the bending portion increases with the angle through which the bending portion has to be bent (flexed).

Particularly in recent years, the use of endoscopes with a substantial overall length that covers a long distance from the manipulating section to the bending portion, as exemplified by endoscopes that are dedicated to the lower digestive tract or industrial endoscopes, is increasing and an even greater burden is imposed on operators who are manipulating those types of endoscopes.

To solve this problem, Japanese Patent NOs. 3017769 and 3030129 have disclosed endoscopes in which the manipulating section is provided with electrical motors for pulling wires (by rotating pulleys) and switches such as joysticks for driving (rotating in a forward and a reverse direction) the electrical motors, so that the wires are pulled not by human power but by the motors' power to bend the bending portion.

These endoscopes have the advantage that the operator requires no great force to bend the bending portion.

However, these endoscopes entirely depend on the motors' power to bend the bending portion. Hence, a power failure or a machine trouble makes the operation to bend the bending portion impossible. If these endoscopes become impossible to manipulate while the insertion section is being inserted into the human body and with the bending portion being bent, the bent portion may get stuck in the human body and cannot be pulled out; if it is forcibly pulled out of the human body, the human body might be accidentally perforated or otherwise damaged.

Another problem arises from the very mechanism of those endoscopes; a time lag occurs between the manipulation by the operator and the pulling by the motor, namely, the bending of the bending portion, which introduces difficulty in subtly manipulating the bending portion to bend.

Furthermore, if the bending portion of an endoscope, after it is pressed into a body cavity, is forcibly bent, it may potentially damage the human body. However, with an endoscope of such a type that the bending portion is to be bent by the power of an electrical motor and the like, the force being actually applied to the bending portion (the counter force the human body is exerting on the bending portion) is not transmitted to the operator. Hence, the operator who has to feel for the counter force being exerted on the bending section to ensure that it will not damage the human body is unable to do this while manipulating the endoscope, which may potentially lead to an accident.

Under these circumstances, there has been proposed an endoscope that requires only a small manipulative force to bend its bending portion and which involves less of the problem with power failures or machine troubles and less of the problem with maneuverability; an example is the endoscope (particularly, an apparatus for manipulating the bending of the endoscope) disclosed in JP 2005-28018 A.

This endoscope does not entirely depend upon the power of a motor to bend its bending portion; instead, it detects the amounts in which (manipulating) wires are pulled by the action of the manipulating means and in accordance with the detected amount, an assist motor is operated to assist in the pulling of an associated wire by a force less than what is required to increase the flexing of the bending portion. According to the disclosures in JP 2005-28018 A, the endoscope uses rotatable knobs as the means of manipulating the bending portion and the amounts in which the wires are pulled are detected by detecting the angles through which the knobs are rotated (the angles through which gears rotate in response to the turning of the knobs).

In accordance of the preamble of claim 1, US-A-5,469,840 discloses an endoscope in which the sensing means is positioned between a driving motor (auxiliary means) and a sprocket which is coupled via a chain to an operation wire (pulling means).

SUMMARY OF THE INVENTION

In the endoscope disclosed in JP 2005-28018 A, the bending of the bending portion is basically achieved by the operator who controls the manipulating means to pull the wires and the assist motor simply assists in the operator's pulling of the wires.

Thus, even if a power failure or a machine trouble in a motor occurs after the insertion section of the endoscope has been inserted into the human body and with the bending portion having been bent, the manipulating means can be controlled to perform a suitable action such as straightening the bent portion, thus allowing the insertion section to be safely pulled out of the human body.

In addition, the bending operation is performed by pulling the wires by the manipulating means, so the bending portion can be bent without any delay after a command for bending is entered by the operator. As a further advantage, the counter force the human body exerts on the bending portion is transmitted via the wires, so the operator can manipulate the endoscope while feeling for the counter force being exerted on the bending portion and this allows for safe diagnosis.

As noted above, the endoscope of JP 2005-28018 A uses the assist motor to assist in the pulling of a wire according to the amount in which it is pulled by the action of the manipulating means (or the angle through which the manipulating means is rotated).

Specifically, if the wire is pulled by a small amount, the output power of the assist motor is held to a small enough value (i.e., the amount of assist is reduced) and as the wire is pulled by an increasingly greater amount, the output power of the assist motor is increased (i.e., the amount of assist is increased) in steps.

As the endoscope is used for an extended period, various parts of it will inevitably deteriorate. As a result, the wire pulling force required to bend the bending portion gradually increases with time and so does the manipulative force required to bend the bending portion.

This means that even if the assist motor is used to assist in wire pulling in accordance with the amount by which the wire is pulled, deterioration due to aging will prevent the assist motor from providing a good assist in traction, hence, manipulative force, with the result that a very large manipulative force is occasionally required even if the bending portion needs to be bent by a small amount.

Depending on the condition of the site being examined or the state of the endoscope at the site being examined, a large manipulative force is sometimes required even if the amount by which the bending portion needs to be bent, namely, the amount by which a wire is pulled is small.

However, if the amount of wire pulling, namely, the amount of bending is small, the assist motor used in the endoscope of JP 2005-28018 A produces only a small output power and hence is incapable of providing a good assist in manipulative force.

In short, the conventional endoscope having an auxiliary means that provides an assist in the operator's manipulative force is free from potential damage to the human body or other risks that may result from a machine trouble or other accidents and the operator who is manipulating it can feel for the counter force as it is exerted on the bending portion; an additional advantage of this endoscope is that it reduces the burden on the operator. On the other hand, this endoscope sometimes fails to provide a good assist in its manipulation by the operator (as regards the force required to manipulate it) to accommodate its deterioration due to aging, as well as the condition of the site being examined or the state of the endoscope at the site being examined.

The present invention has been accomplished under these circumstances and has as its object providing an endoscope having the following features: even if motors and the like that assist in wire pulling stop running due to a power failure, a machine trouble and the like, the bending of the bending portion can be controlled to ensure that the insertion section of the endoscope is safely extracted from the human body or the like; the operator can perform subtle manipulation of the endoscope while feeling the manipulating means for the counter force being exerted on it by the site within the human body or the like that is under examination and without any delay in the bending of the bending portion in response to a command for bending that is entered by the operator; an appropriate and consistent assist can be provided for the pulling by the pulling means which pulls the bending portion, namely, the operator's manipulative force, to accommodate the condition of the endoscope that has aged or has been used in a hostile situation, the condition of the site under examination such as a body cavity, or even the state of the endoscope at the site under examination.

In order to attain the above described object, the present invention provides an endoscope comprising the features of claim 1.

Preferred embodiments are defined by the dependent claims.

Preferably, the manipulating means is turned to cause the pulling means to pull the bending portion, and the sensing means is a torque sensor that senses a torque applied to the manipulating means. And, preferably, the control means controls drive of the auxiliary means in such a way that in accordance with the manipulative force applied to the manipulating means and sensed by the sensing means, it assists in the pulling of the bending portion by the pulling means with a force that accounts for a predetermined proportion of the applied manipulative force.

In addition, preferably, the control means controls drive of the auxiliary means in such a way that, if the manipulative force applied to the manipulating means and sensed by the sensing means is equal to or less than a predetermined value, the pulling of the bending portion by the pulling means is not assisted, and the control means controls drive of the auxiliary means in such a way that, if the manipulative force applied to the manipulating means and sensed by the sensing means is equal to or less than a predetermined value, the pulling of the bending portion by the pulling means is associated with an auxiliary force that accounts for a smaller proportion of the applied manipulative force than in a case where the applied manipulative force exceeds the predetermined value.

Preferably, the control means controls drive of the auxiliary means in such a way that, if the manipulative force applied to the manipulating means and sensed by the sensing means exceeds a predetermined value, the pulling of the bending portion by the pulling means is not assisted. And, preferably, the control means controls drive of the auxiliary means in such a way that, if the manipulative force applied to the manipulating means and sensed by the sensing means exceeds a predetermined value, the pulling of the bending portion by the pulling means is assisted with an auxiliary force same as what is produced in a case where the applied manipulative force is of the predetermined value.

It is preferable that the endoscope further comprises a rotating shaft that rotates integrally with the manipulating means to transmit the rotation of the manipulating means to the pulling means, wherein the auxiliary means engages directly or indirectly with the rotating shaft to assist in the pulling of the bending portion by the pulling means and the sensing means senses the manipulative force applied to the manipulating means, by sensing a rotational force on the rotating shaft in a position that is closer to the manipulating means than to a position of engagement of the auxiliary means.

Having the above-described structure, the endoscope of the present invention is such that its bending portion is basically bent by the pulling means such as wires that link the manipulating means to the bending portion and that, in addition, in accordance with the force applied to the manipulating means by the operator who is manipulating the endoscope, the auxiliary means such as motors assist in the pulling of the bending portion by the pulling means.

Thus, even if a power failure or a machine trouble such as motor trouble occurs during the use of the endoscope of the present invention, the operator can appropriately manipulate the bending portion by the manipulating means to extract the insertion section of the endoscope in a safe manner. Since the bending portion is basically bent by the pulling action of the pulling means in response to the manipulation by the manipulating means, the operator can perform the bending operation while feeling for the counter force that is being exerted on the bending portion by a site in the human body or the like under examination; hence, damage to the human body from an accident such as perforation can be effectively prevented to assure safe operation of the endoscope. Furthermore, unlike in the case of depending solely on motors to bend it, the bending portion will bend with little delay in response to a bending action of the manipulating means and this enables the operator to manipulate the endoscope in a very subtle manner.

Another feature of the endoscope of the present invention is that in accordance with the manipulative force applied to the manipulating means, the traction by the pulling means is assisted, namely, a force required to manipulate the manipulating means is furnished. Thus, only a small manipulative force suffices to bend the bending portion and the burden that is imposed on the operator to manipulate the bending portion can be significantly reduced.

What is more, the traction by the pulling means is assisted not in accordance with the amount of traction by the pulling means such as wires (i.e., the amount of manipulation required to bend the bending portion) but in accordance with the manipulative force exerted on the manipulating means. Hence, even in a case where a greater manipulative force is required to bend the bending portion for such a reason that the wires and the like have developed an increased force of friction due to such a phenomenon as deterioration from aging or use in a hostile situation or in such a case that due to the condition of the site under examination or the state of the endoscope at the site under examination, a great force is required to bend the bending portion even by a small amount, the operator may apply a greater force to the manipulating means, whereupon the traction by the pulling means is accordingly assisted; as a result, in accordance with the force that is required to bend the bending portion, the traction, or the manipulation of the manipulating means (the force required to manipulate it), can be assisted to provide a supplemental force required to manipulate it.

Thus, with the endoscope of the present invention, the traction by the pulling means that bends the bending portion can be assisted in a consistent and appropriate manner in accordance with the required manipulative force that depends on such factors as the state of the endoscope and the condition of the site under examination and, hence, the burden that is imposed on the operator to bend the bending portion, in particular, the force required to bend it can be sufficiently reduced to provide ease in the bending operation.

BRIEF DESCRIPTION OF THE DRAWINGS

FIG. 1 is a perspective view showing in concept an example of the endoscope of the present invention.
FIG. 2 is a diagram showing in concept the bending mechanism of the angle portion of the endoscope shown in FIG. 1.
FIG. 3 is a diagrammatic view of an example of the angle portion that can be used with the endoscope of the present invention.
FIG. 4 is a graph illustrating how the assisting in the bending of the angle portion of the endoscope shown in FIG. 1 may be controlled.
FIGS. 5A and 5B are block diagrams for two examples of a system for controlling the assisting in the bending of the angle portion of the endoscope shown in FIG. 1.
FIGS. 6A to 6E are graphs illustrating various modes for assisting in the bending of the angle portion of the endoscope of the present invention.
FIG. 7 is a diagrammatic view of an example of the bend manipulating section of the endoscope of the present invention.
FIG. 8 is a diagrammatic view of another example of the bend manipulating section of the endoscope of the present invention.

DETAILED DESCRIPTION OF THE INVENTION

On the following pages, the endoscope of the present invention is described in detail with reference to the preferred embodiments depicted in the accompanying drawings.

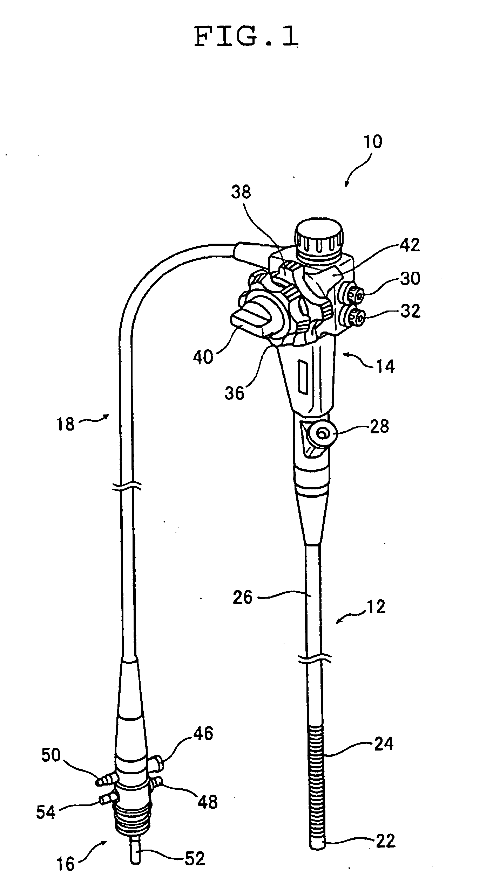

FIG. 1 is a diagrammatic view showing an example of the endoscope of the present invention.

The endoscope generally indicated by 10 in FIG. 1 is inserted into a site such as a body cavity (e.g. the digestive tract or the ears, nose or larynx) to be examined and is typically used to observe that site, take a still or moving picture of the site, or even collect a tissue of the site.

Except that it has an assist mechanism (auxiliary mechanism) to be described later which serves to assist in the bending of an angle portion 24, the endoscope 10 is basically the same as a known conventional endoscope (endoscopic apparatus) in that it is composed of an insertion section 12, a manipulating section 14, a connector 16, and a universal cord 18 as in the ordinary endoscope.

The insertion section 12 is an elongated site that is to be inserted into a site such as a body cavity to be examined and it has a distal end portion 22 at the distal end (the distal end which is to be inserted and away from the manipulating section 14), an angle portion 24, and a flexible portion 26.

The distal end portion 22 is typically equipped with a lighting glass for performing illumination with a light guide, air insufflation and water feeding nozzles through which air is typically withdraw from the site under examination or air or water is fed to the site, and a forceps port through which forceps are inserted into the site under examination, typically to collect a tissue. If the endoscope 10 is an electronic scope for imaging the site under examination using an image sensor such as a CCD sensor (if it has such a function), an imaging objective lens, a CCD sensor and the like are attached to the distal end portion 22; if the endoscope 10 is a fiberscope with which the site under examination can be directly observed (if it has such a function), a viewing lens, a viewing window and the like are attached to the distal end portion 22.

The angle portion (bending portion) 24 is a region which is bent up and down or from right to left and vice versa (in four orthogonal directions) by manipulation of the manipulating section 14 in order to insert the distal end portion 22 into a desired position or locate it in a desired position. The angle portion 24 is bent by manipulating the knobs, to be described later, on the manipulating section 14 (bend manipulating means = a LR knob 36 and an UD knob 38). Details of this point will be given later.

The flexible portion 26 is a site that connects the distal end portion 22 and the angle portion 24 to the manipulating section 14; it is an elongated member that is sufficiently flexible to allow the distal end portion 22 to be inserted into the site to be examined. The flexible portion 26 (and the angle portion 24) typically accommodates a forceps channel (tube) into which forceps are to be inserted, air insufflation and water feeding channels that are to be connected to the air insufflation and water feeding nozzles, a light guide for illuminating the site to be examined, and a cable for imaging the site under examination (a viewing image guide).

The manipulating section 14 is a site for manipulating the endoscope 10.

As in the ordinary endoscope, the manipulating section 14 is typically fitted with a forceps port 28 through which forceps are to be inserted, a suction button 30 for performing suction through the air insufflation and water feeding nozzles at the distal end portion 22, as well as an air insufflation/water feeding button 32 for performing air insufflation and water feeding through the air insufflation and water feeding nozzles at the distal end portion 22. If the endoscope 10 is an electronic scope, a variety of switches such as a zooming switch and an imaging switch (which constitute manipulating means for use in imaging applications) are attached to the manipulating section 14; if the endoscope 10 is a fiberscope, an eyepiece and the like are attached to the manipulating section 14.

As mentioned before, the manipulating section 14 is furnished with a manipulating means for bending the angle portion 24 of the insertion section 12.

Specifically, a LR knob (left/right knob) 36 for bending the angle portion 24 to the left or right and an UD knob (up/down knob) 38 for bending the angle portion 24 up and down in directions perpendicular to the aforementioned left and right directions are provided in the manipulating section 14. The endoscope 10 is similar to various other endoscopes in that the LR knob 36 is turned to bend (flex) the angle portion 24 of the insertion section 12 to left or right whereas the UD knob 38 is turned to bend the angle portion 24 up and down. Details of the mechanism by which the angle portion 24 is bent will be given later.

The manipulating section 14 is also fitted with a LR fixing knob 40 which fixes the angle portion 24 (therefore, the LR knob 36) as it is bent to either left or right, as well as an UD fixing knob 42 which fixes the angle portion 24 (therefore, the UD knob 38) as it is bent.either up or down.

The connector (light guide (LG) connector) 16 is a part that connects the endoscope 10 to water feeding means, air insufflation means, suction means and the like in a facility where the endoscope is to be used; the connector 16 is typically fitted with a water feeding connector 46 for connecting the endoscope 10 to the water feeding (water supplying) means in the facility, a ventilating connector 48 for connecting the endoscope 10 to the air insufflation means in the facility, and a suction connector 50 for connecting the endoscope 10 to the suction means in the facility.

The connector 16 is also furnished with a LG rod 52 for connecting the illuminating light guide to a light source, and an S terminal 54 to which an S cord is connected to provide a path for diverting the leakage current to the endoscope 10 in the process of using a RF treating tool (a so-called electrical scalpel such as an electrical snare or knife).

If the endoscope 10 is an electronic scope, the connector 16 is also connected to a video connector for connecting a video processor, a monitor and the like to the endoscope 10.

The universal cord (LG flexible section) 18 is a site that connects the connector 16 to the manipulating section 14.

The lightguide, air insufflation/water feeding channels, etc. extending from the connector 16 pass through the universal cord 18 to be connected to the manipulating section 14, from which they extend to pass through the flexible portion 26 of the insertion section 12 to be connected to the distal end portion 22 as mentioned before.

As mentioned before, the angle portion 24 of the insertion section 12 is bent to the left or right by means of the LR knob 36 on the manipulating section 14 whereas it is bent up and down by means of the UD knob 38.

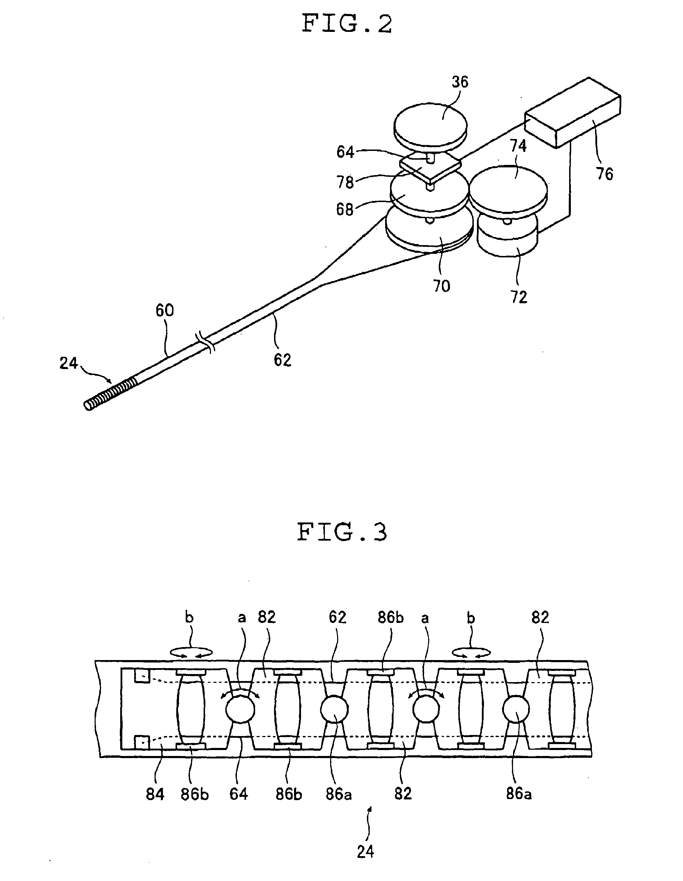

FIG. 2 shows in concept the bending mechanism for use with the angle portion 24.

FIG. 2 shows the mechanism by which the LR knob 36 is turned to bend the angle portion 24 to either left or right.

In the illustrated case, the angle portion 24 is similar to the angle portion of a known endoscope in that it has a number of circular rings connected together. To the inner right and left sides of each of the rings that compose the angle portion 24 (i.e., in specified positions on the diameter across the inner surface of each ring), there are connected wires (angle wires) 60 and 62 by means of which the angle portion 24 is pulled to bend in the right and the left direction.

Either of the two wires 60 and 62 is pulled by turning the LR knob 36 and, as a result, the angle portion 24 is bent in such a way that the side on which traction has been exerted (the pulled side) contracts.

FIG. 3 is a diagrammatic view for a specific example of the angle portion 24.

Note that the construction of the angle portion 24 in the endoscope of the present invention is by no means limited to the illustrated case and that all designs that are adopted in a variety of endoscopes can be utilized.

In an exemplary case, the illustrated angle portion 24 is composed of nine rings connected together, which consist of eight circular rings 82 and one distal-end ring 84.

The circular rings 82 are each a generally cylindrical member which is slightly curved as seen from the lateral side. The distal-end ring 84 is also a generally cylindrical member, which is located in the angle portion 24 on the closest side to the distal end portion 22.

The circular rings 82 and the distal-end ring 84 are linked by linking pins 86a and linking pins 86b.

The circular rings 82 are arranged in such a way that their curving directions (the directions in which they convex or concave) alternate along the length of the insertion section 12. The linking pin 86a connects two circular rings 82 at their convex centers in such a way that they can swing from right to left and vice versa (in the direction indicated by two-headed arrow a). In contrast, a pair of the linking pins 86b connect two adjacent circular rings 82, as well as the circular ring 82 at the distal end to the distal-end ring 84 at opposing ends of their concave portions in such a way that they can swing up and down (in the direction indicated by two-headed arrow b).

In addition, the linking pins 86a and linking pins 86b are alternately arranged to connect the circular rings 82. Thus, the circular rings 82 are so linked as to be capable of turning in two alternate directions, one being vertical and other horizontal.

The wires 60 and 62 for bending the angle portion 24 to right and left are spaced apart in the horizontal direction as they are passed through the circular rings 82; in one example, the distal end of the wire 60 is secured to the inner right side of the distal-end ring 84 whereas the distal end of the wire 62 is secured to the inner left side of the distal-end ring 84. Although not shown, two (angle) wires for bending the angle portion 24 up and down are spaced apart in the vertical direction (perpendicular to the paper on which FIG. 3 is drawn) as they are passed through the circular rings 82 in such a way that the distal end of one wire is secured to the inner upper side of the distal-end ring 84 whereas the distal end of the other wire is secured to the inner lower side of the distal-end ring 84.

As mentioned before, the manipulating section 14 is fitted with the LR knob 36 for bending the angle portion 24 to either left or right.

Having a rotating shaft 64 fixed thereto, the LR knob 36 is axially supported by this rotating shaft 64 to be capable of rotation. A pulley 70 is fixed to the lower end of the rotating shaft 64 and a gear 68 is fixed between the LR knob 36 on the rotating shaft 64 and the pulley 70. Secured to the groove in the pulley 70 is the opposite end of each of the wires 60 and 62 which are guided by a guide member (not shown) to be connected to the aforementioned angle portion 24. In the illustrated case, the pulley 70 as well as the wires 60 and 62 constitute the pulling means that pulls the angle portion 24 to bend it.

The gear 68 meshes with a gear 74 that is fixed to the rotating shaft of a motor 72 (say, a DC motor). Provided between the LR knob 36 on the rotating shaft 64 and the gear 68 is a torque sensor 78 for sensing the torque the LR knob 36 has exerted on the rotating shaft 64 as it turns, namely, the manipulative force applied to the LR knob 36. Furthermore, control means 76 for controlling the drive of the motor 72 is connected to the torque sensor 78 and the motor 72. The motor 72 is an assist motor which, when the wires 60 and 62 are pulled to bend the angle portion 24, assists in the pulling of those wires, namely, the bending of the angle portion 24; the motor 72 is the auxiliary means in the present invention.

As mentioned before, the manipulating section 14 of the endoscope 10 has not only the LR knob 36 but also the UD knob 38 for bending the angle portion 24 up and down.

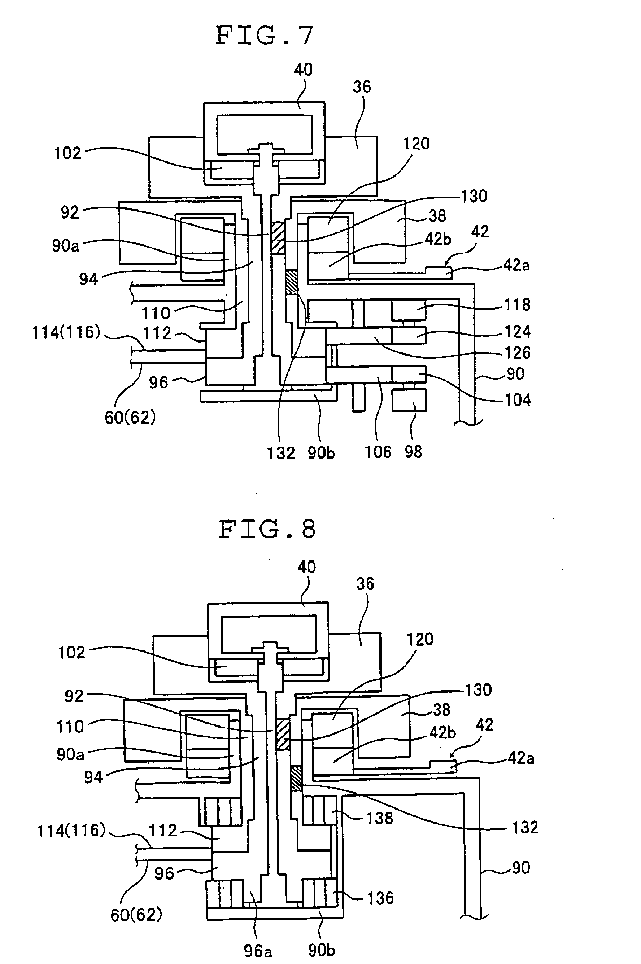

In order to get the angle portion 24 to bend up and down by means of the UD knob 38, the same mechanism as the one that comprises the LR knob 36, torque sensor 78, gear 68 and the pulley 70 is built in the manipulating section 14 coaxially with the rotating shaft 64 (in alignment with respect to the center of rotation) by a known means such as a sheathed tube structure of the type described in JP 2005-28018 A and shown in FIGS. 7 and 8 to be discussed later; this mechanism engages with and connects to the same mechanism as the one consisting of the gear 74 and the motor 72, as well as the control means 76.

As also mentioned before, the wires 60 and 62 associated with the LR knob 36 are connected in horizontal directions; in contrast, the wires that are to be pulled by turning the UD knob 38 are connected in vertical directions on the inner surfaces of the rings that compose the angle portion 24 (at points on the inner surfaces of the rings across the diameter perpendicular to the diameter of the inner surfaces of the rings at which the wires 60 and 62 are connected).

The illustrated endoscope 10 is operated in the following manner. When the operator turns the LR knob 36, the rotating shaft 64 and the pulley 70 (as well as the gear 68) are rotated. Depending on the direction in which the pulley 70 is rotated, either the wire 60 or 62 is pulled, causing the angle portion 24 to be pulled accordingly, whereupon the angle portion 24 is bent (flexed) with the pulled wire being contracted.

Specifically, as mentioned before, the circular rings 82 in the angle portion 24 are linked together in such a way that they are free to rotate in two alternate directions, one being horizontal and the other vertical. Hence, by turning the LR knob 36 so as to rotate the pulley 70 to which the wires 60 and 62 are linked, either one of the wires 60 and 62 is advanced while the other wire is pulled depending on the direction in which the pulley 70 rotates, whereupon the angle portion 24 is bent to either left or right. Similarly, by turning the UD knob 38 so as to rotate the pulley to which the up wire and the down wire are linked, either one of these wires is advanced while the other wire is pulled depending on the direction in which the pulley rotates, whereupon the angle portion 24 is bent either up or down.

The greater the amount by which the wire 60 or 62 is pulled, namely, the greater the amount of rotation of the pulley 70, the greater the degree by which the angle portion 24 is bent. Therefore, the amount by which the angle portion 24 is bent can be adjusted by controlling the amount by which the LR knob 36 is turned.

As mentioned before, the endoscope 10 has not only the mechanism by which the angle portion 24 is bent to right and left by means of the LR knob 36 but also the mechanism by which the angle portion 24 is bent up and down by means of the UD knob 38.

Therefore, by manipulating the LR knob 36 and the UD knob 38, the operator can bend the angle portion 24 by a desired amount (less than the limit of bending) in any direction that is a combination of the right or left direction and the up or down direction, whereby the operator can observe or take a picture of the site under examination, or collect a tissue from that site.

Note that the mechanism by which the angle portion of the endoscope of the present invention is bent is by no means limited to the illustrated case and one may utilize various mechanisms (means) for bending the angle portion that employ pulling means such as wires and which are adopted in various types of endoscopes.

Here the rotating shaft 64 that is rotated by the LR knob 36 is fitted with the torque sensor 78. This torque sensor 78 senses the manipulative force (rotating torque) the operator has exerted on the LR knob 36 to bend the angle portion 24.

The gear 68 is fixed to the rotating shaft 64 and this gear 68 meshes with the gear 74 which is fixed to the rotating shaft of the motor 72.

The torque sensor 78 and the motor 72 in turn are connected to the control means 76 for controlling the drive of the motor 72.

The control means 76 acquires from the torque sensor 78 the information about the torque exerted on the LR knob 36. The control means 76 drives the motor 72 in such a way that an amount of torque that accounts for a specified proportion of the torque (manipulative force) the operator has exerted upon the LR knob 36 is applied to the rotating shaft 64.

Consequently, a certain percentage of the force that is required to turn the LR knob 36 in order to bend the angle portion 24 (i.e., the force required to pull the wire) is supplied by the motor 72, so that a small manipulative force suffices to turn the LR knob 36, namely, to bend the angle portion 24.

As will be apparent from the foregoing description, in order to ensure that only the manipulative force exerted on the LR knob 36 is detected appropriately, the torque exerted on the LR knob 36 has to be sensed by the torque sensor 78 in a position somewhere between the motor 72 and the LR knob 36 (upstream of the motor 72 in the direction in which the manipulative force is transmitted from the LR knob 36 toward the motor 72).

With the endoscope, it is generally held that the greater the amount by which the angle portion need be bent, namely, the greater the amount by which the manipulating means for bending the angle portion is manipulated (i.e., the amount of rotation of the knob), the greater the force that is required to perform the bending operation.

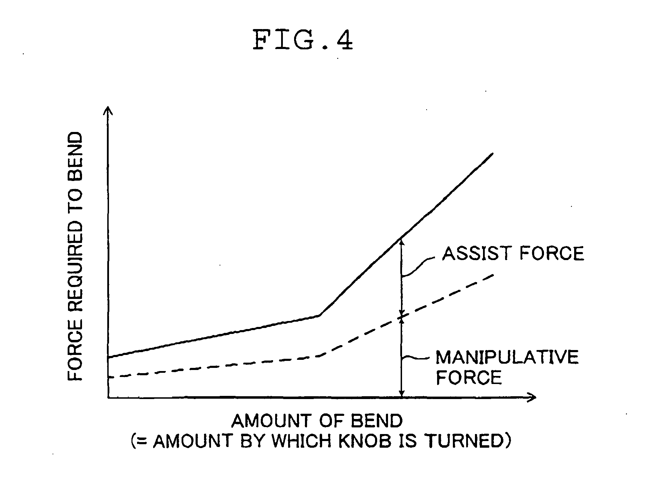

Assume here, for example, that the force (torque) that is required to bend the angle portion 24 and the amount of bend in the angle portion 24 are related by the solid line in FIG. 4.

In one example of this case, the control means 76 drives the motor 72 in such a way that the torque applied to the rotating shaft 64 is always equal (100%) to the torque the operator exerted on the LR knob 36. In other words, when the operator exerts a torque on the LR knob 36 that is indicated by the dashed line in FIG. 4, the motor 72 exerts the same amount of torque on the rotating shaft 64. Therefore, if the operator manipulates the LR knob 36 with a force of 50, the motor 72 applies the same amount of force (= 50) to the rotating shaft 64, whereupon the bending operation is performed by a force which amounts to a total of 100. In other words, while the force indicated by the solid line is required to bend the angle portion 24, one half of that force which is indicated by the dashed line is supplied by the motor 72 to assist in the turning of the LR knob 36.

Therefore, the manipulative force required by the operator to bend the angle portion 24 is equal to the torque indicated by the solid line in FIG. 4 minus the torque (assist force) indicated by the dashed line, which means that one half of the required manipulative force (torque) suffices to turn the LR knob 36; as a result, the angle portion 24 can be bent with a very small force.

As mentioned before, the bending of the angle portion 24 is an operation that requires a great force and imposes a substantial burden on the operator who manipulates the endoscope. With a view to solving this problem, Japanese Patent NOs. 3017769 and 3030129 disclose endoscopes that depend on the power of motors to bend the angle portion 24. However, with these endoscopes, if a machine trouble or a power failure occurs, the angle portion cannot be manipulated to bend, potentially causing an accident such as damage to the human body; a further possibility is the operator's inability to feel for the counter force being exerted on the angle portion, thus failing to perform a safe operation for bending the angle portion.

These inconveniences can be eliminated by the endoscope described in JP 2005-28018 A which employs an assist motor that assists in the pulling of wires in accordance with the amount by which the wires are pulled to pull and bend the angle portion (i.e., the amount by which either of the knobs is turned to bend the angle portion); the operation for bending the angle portion can be performed even if a power failure or other accident occurs and the operator can perform the bending operation while feeling for the counter force that is being exerted on the angle portion. However, this endoscope which assists in the bending of the angle portion in accordance with the amount of pulling of the wires has the disadvantage that if the force required by the bending operation is increased on account of aging, deterioration or other causes, no suitable assisting that allows for this increase cannot be performed and, what is more, even if the condition of the site under examination, the state of the endoscope at the site under examination or other factors is such that a great manipulative force is required notwithstanding the small amount of bending that is to be achieved, no suitable assisting that allows for this situation cannot be performed, either.

In contrast, the endoscope of the present invention bends the angle portion 24 by pulling the wires 60 and 62 primarily by manipulating (turning) the LR knob 36 and other members of the manipulating means; the pulling of the wires 60 and 62, namely, the bending of the angle portion 24 is assisted by the motor 72 and other members of the auxiliary means in accordance with the torque by which the operator turned the LR knob 36 (the manipulative force applied to the manipulating means).

Therefore, the endoscope 10 of the present invention has such an advantage that even if a power failure or a machine trouble such as one in the motor 72 occurs, the LR knob 36 can be appropriately turned to control the bending of the angle portion 24 so that the insertion section 12 can be extracted in a safe manner. In addition, since the bending of the angle portion 24 is primarily performed by pulling the wires 60 and 62 with the aid of the LR knob 36 and other members of the manipulating means, the operator can perform the operation of bending the angle portion 24 while feeling for the counter force the site under examination is exerting on it; as a result, an accident such as perforation can be effectively prevented to permit a safe operation for bending the angle portion 24. What is more, the manipulation of the LR knob 36 and other members of the manipulating means allows the angle portion 24 to be bent rapidly enough to enable a subtle operation for bending it.

The other feature of the endoscope of the present invention is that the assisting in bending by the motor 72 is performed not in accordance with the amount of pulling of the wires (the amount of turning either of the knobs, or the amount of manipulation), but in accordance with the torque (manipulative force) applied to the LR knob 36 and other members of the manipulating means. Therefore, even in the case where the manipulative force required by the bending operation is increased due, for example, to aging or deterioration that depends on the situation in which the endoscope has been used (e.g., an increase in the force of friction that develops on the wires 60 and 62) or in the case where the condition of the site under examination, the state of the endoscope at the site under examination or other factors is such that a great manipulative force is required notwithstanding the small amount of bending that is to be achieved, an increase in the manipulative force that is being exerted by the operator on the LR knob 36 and other members of the manipulating means leads to a corresponding amount of assisting in the pulling of the wires 60 and 62 or the like; thus, in accordance with the manipulative force required to bend the angle portion 24, there is achieved consistent assisting in the manipulation of the LR knob 36 and other members of the manipulating means (the force required for their manipulation is supplied consistently).

In short, with the endoscope of the present invention, the pulling means can be assisted in pulling the angle portion 24 in a consistent and appropriate manner in accordance with the manipulative force required to bend the angle portion 24 and, therefore, the burden on the operator who is engaged in bending the angle portion 24 is reduced consistently at all times to provide ease in performing the operation of bending the angle portion 24.

The foregoing embodiment relates to an exemplary case where an amount of torque equal to the one that is applied to the LR knob 36 is produced by the motor 72 to assist in the manipulation of the LR knob 36, or the bending of the angle portion 24. However, this is not the sole case of the present invention and the bending of the angle portion 24 may be assisted in various proportions, as exemplified by such a design that 30% or 80% of the torque that is applied to the LR knob 36 is supplied as a supplementary force by the motor 72.

In addition, as mentioned before, the manipulative force required to bend the angle,portion 24 generally increases with the amount by which it needs to be bent, so the proportion of assisting by the motor 72 may be increased continuously or stepwise in accordance as the manipulative force exerted on the LR knob 36 increases.

In the foregoing embodiment, given a certain amount of manipulative force (torque) that is applied to the LR knob 36, a specified proportion of that torque is supplied as a supplemental force by the motor 72 but this is not the sole case of the present invention and various other methods may be employed to control the auxiliary force.

In an exemplary method, the auxiliary force is controlled such that the motor 72 assists in the bending of the angle portion 24 by supplying a supplemental force that is equal to the manipulative force exerted by the operator as multiplied by a predetermined coefficient.

Write, for example, W for the force (load) required to bend the angle portion 24, F for the force by which the operator manipulates the LR knob 36, and T for the auxiliary force the motor 72 applies to the rotating shaft 64 (LR knob 36); then, these three forces in the endoscope 10 of the present invention are related by:

$$W = T + F.$$

If the coefficient (gain) of assisting is written as k, the drive of the motor 72 is controlled in such a way that T = kF.

In this case,

$$F(1+k) = W$$

$$F = (1/(1+k))W;$$

thus, the manipulative force F that must be exerted by the operator can accordingly be reduced by (1+k) times the force that is actually required to bend the angle portion 24. If coefficient k is set at unity, the case under consideration is equivalent to the case where 50% of the manipulative force is supplied as an auxiliary force by the motor 72.

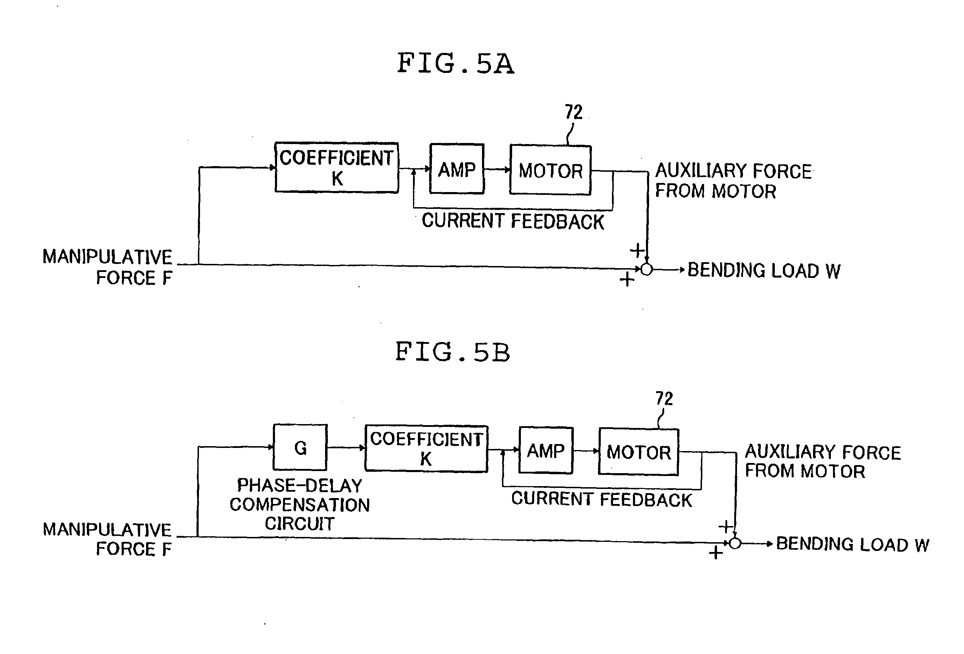

FIG. 5A is a block diagram showing an exemplary system for controlling the auxiliary force in the manner just described above.

With the illustrated control system, if the operator manipulates the LR knob 36, the manipulative force F (manipulative torque) is multiplied by the coefficient k and the product is sent to the Amp, where it is amplified to drive the motor 72. The auxiliary force produced by the motor 72 (motor-assist torque) is added to the manipulative force F exerted upon the LR knob 36 to yield the bending load W.

Another feature of the illustrated control system is that the motor 72 (DC motor) is subjected to feedback control (current feedback) so as to produce a constant current value; because of this feature, the motor 72 is so controlled as to output a constant amount of torque in response to a signal input from the Amp (i.e., an assist command value).

The control system under consideration may incorporate a phase-delay compensation circuit G as shown in FIG. 5B.

In the case of an operation which is performed at high frequency as when the LR knob 36 is turned in a forward and a reverse direction at very short intervals, or in the case of an operation (particularly one at high frequency) on which noise is superposed, the control system may sometimes oscillate. In particular, the angle portion 24 in or near its central (straight) position with less bending has a greater chance for oscillation due to such effects as backlash. If the control system oscillates, the motor 72 is running irrespective of the manipulation of the LR knob 36 and other members of the manipulating means and there occurs such an inconvenience as a tendency of the motor 72 to bend the angle portion 24 in a direction reverse to that of the manipulating load.

In order to avoid such inconveniences, a phase-delay compensation circuit G such as a low-pass filter for removing the high-frequency component is preferably incorporated in the control system to prevent it from oscillating.

In the foregoing examples, given a certain amount of manipulative force, an auxiliary force that is equal to a predetermined constant proportion of the manipulative force is supplied by the motor 72.

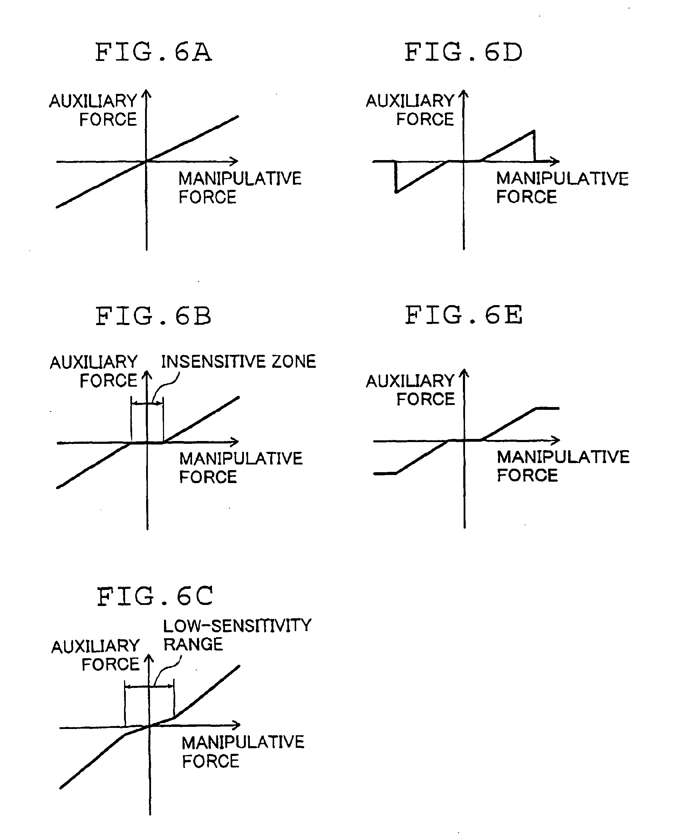

Specifically, as shown in FIG. 6A, with a certain amount of manipulative force (input) applied to the LR knob 36, the motor 72 assists in the bending of the angle portion 24 by applying an auxiliary force (as output power from the motor 72) in such a way that it increases at a predetermined gradient.

However, this is not the sole case of the present invention and there may be employed various designs (variations) to assist in the bending of the angle portion 24.

For instance, as mentioned before, the angle portion 24 in or near its central position with less bending has a greater chance for oscillation. In addition, in or near the central position, the angle portion 24 requires a very small amount of force to be bent, which means that the motor 72 has no need to assist in the bending of the angle portion 24.

Therefore, if there is applied only a small amount of manipulative force to the LR knob 36, the motor 72 need not assist in the bending of the angle portion 24. Thus, as shown in FIG. 6B, a region that may be called "an insensitive zone" may be provided in that range where only a small amount of manipulative force is applied and no assisting is performed in this insensitive zone but when a manipulative force beyond that insensitive zone is applied, assisting is performed in accordance with the applied manipulative force.

Alternatively, a region where the manipulative force applied is smaller than a predetermined value may be assigned as a region where an auxiliary force is produced in a smaller proportion to the applied manipulative force than in the other regions. For instance, the insensitive zone shown in FIG. 6B may be replaced by a low-sensitivity range as shown in FIG. 6C which is the region where the manipulative force applied is smaller than a predetermined value and where the response (sensitivity) to the applied manipulative force is low; in this low-sensitivity range, the proportion of the auxiliary force that is produced by the motor 72 as relative to the applied manipulative force may be adjusted to be smaller than in the other regions (where the applied manipulative force exceeds the predetermined value).

Conversely, if the manipulative force applied to the LR knob 36 becomes very large, there is high possibility that the angle portion 24 (distal-end portion 22) may have gotten stuck within the human body or it may be exerting a strong compressive force within the human body. If, in this case, the angle portion 24 is further forced to bend, an accident such as perforation might even occur to damage the human body.

Therefore, if the manipulative force being exerted on the LR knob 36 exceeds a predetermined value, the assisting by the motor 72 may be turned off (suspended) as shown in FIG. 6D.

Alternatively, as shown in FIG. 6E, if the manipulative force being applied to the LR knob 36 exceeds a predetermined value, the auxiliary force produced by the motor 72 may not be further increased but held constant. In other words, depending on the manipulative force applied to the LR knob 36, a limit may be provided for the auxiliary force that is produced by the motor 72.

In the examples shown in FIGS. 6D and 6E, an insensitive zone is provided as in the case shown in FIG. 6B but providing such an insensitive zone is not the sole case of the embodiment where assisting by the motor 72 is turned off if a manipulative force greater than a predetermined value is applied and of the design where a limit is provided for the auxiliary force that is produced by the motor 72. Specifically, each of the designs shown in FIGS. 6D and 6E may be so modified that until a predetermined value of manipulative force is applied, the motor 72 produces an auxiliary force in a predetermined constant proportion, as shown in FIG. 6A or, alternatively, they may be so modified that a region where the manipulative force applied is less than a predetermined value is assigned as a low-sensitivity range where the proportion of the auxiliary force relative to the manipulative force is smaller than in the other regions, as shown in FIG. 6C.

In addition to these modifications, various designs can also be utilized to provide a supplemental bending force relative to the manipulative force.

For example, the designs that are shown in FIGS. 6A and 6B may be combined to provide the following modification: an insensitive zone remains effective until a first value of manipulative force is applied; a low-sensitive range takes over when the first value of manipulative force is exceeded and remains effective until a second, greater value of manipulative force is applied; and if the second value of manipulative force is exceeded, an even stronger auxiliary force may be applied. Alternatively, the designs shown in FIGS. 6D and 6E may be combined to provide the following modification: up until a first value of manipulative force is applied, the auxiliary force is gradually increased in accordance with the applied manipulative force; the auxiliary force is held constant up until a second value of manipulative force greater than the first value of manipulative force is applied; and no assisting is performed if the second value of manipulative force is exceeded.

The illustrated endoscope 10 uses the motor 72 as an auxiliary means that assists in the traction by the pulling means for bending the angle portion 24; however, this is not the sole case of the present invention and various other kinds of the auxiliary means may be utilized, as exemplified by solenoids that depend on a fluid pressure or an electromagnetic force to assist in traction.

In addition, the illustrated endoscope 10 uses the torque sensor 78 as the means of sensing the manipulative force applied to the LR knob 36. The torque sensor 78 is not limited in any particular way and various kinds of torque sensor may be utilized, as exemplified by a torque sensor that uses a strain gage or a magnetostrictive torque sensor.

Aside from torque sensors, various other kinds of force sensing means may be utilized as means for sensing the manipulative force applied to the LR knob 36 (manipulating means).

FIG. 7 is a diagrammatic view (diagrammatic sectional view) showing a specific design of the bend manipulating section of the endoscope of the present invention.

It is important that the inside of the manipulating section of the endoscope be sealed against the exterior to have a watertight structure with the aid of an O-ring or the like; however, to clarify the design for bending the angle portion 24 and the design for assisting in its bending, the seal structure is omitted from the illustration. In addition to the watertightness (sealability), the manipulating section of the endoscope is required to meet such conditions as a minimum number of dead ends, good cleanability, and freedom from accidental decomposition.

As mentioned before, the manipulating section 14 of the endoscope 10 has the LR knob 36 for bending the angle portion 24 to either right or left and the UD knob 38 for bending the angle portion 24 either up or down, as well as the LR fixing knob 40 for fixing the LR knob 36 and the UD fixing lever 42 for fixing the UD knob 38.

A housing (immobile) 90 of the manipulating section 14 has a cylindrical insertion portion 90a that is erected to penetrate the wall of the housing 90, and a generally C-shaped fixing portion 90b is provided at the lower end of the insertion portion 90a (in the inner lateral end portion of the housing 90).

The fixing portion 90b has a cylindrical central shaft 92 that is erected past through the insertion portion 90a to project outward from the housing 90.

A linking tube (rotating shaft) 94 is fixed to the underside of the LR knob 36 and a pulley 96 is fixed to the lower end of the linking tube 94. Like the previously mentioned pulley 70, the pulley 96 has the wires 60 and 62 fixed thereto as they are connected to the angle portion 24; the pulley 96 also has a gear formed in such a way that it can be rotated by a LR assist motor 98 (which is hereinafter referred to as a LR motor 98) which is an auxiliary means that assists in the bending of the angle portion 24 to either right or left.

The linking tube 94 is a cylindrical member through which the central shaft 92 passes to axially support it in a rotatable manner. Hence, both the LR knob 36 and the pulley 70 are so designed that they rotate about the central shaft 92 (or its center line), i.e., they are axially supported by the central shaft 92 to be capable of rotation; when the LR knob 36 is turned by the operator, the pulley 70 is rotated by the same amount so that either of the wires 60 and 62 is pulled and the other wire is advanced.

The topside of the LR knob 36 is recessed to receive the lower part of the LR fixing knob 40. Provided on the underside of the LR fixing knob 40 is a LR brake 102 for locking the LR knob 36 against turning.

The LR fixing knob 40 is axially supported by the central shaft 92 to be capable of rotation. On other hand, the LR brake 102 is a cylindrical member through which the central shaft 92 passes to support it in such a manner that it cannot be turned but can make ascent and descent (can move along the length of the central shaft 92, i.e., move up and down).

When the LR fixing knob 40 is turned, the LR brake 102 either ascends or descends, depending on the direction of its turning, by a known means such as a cam mechanism or a screw mechanism.

In its topmost position, the LR brake 102 is fully spaced from the LR knob 36. However, when it descends, the LR brake 102 contacts or depresses the LR knob 36. As mentioned before, the LR brake 102 is supported by the central shaft 92 to be incapable of turning, so if the LR brake 102 depresses the LR knob 36, frictional force develops to hold the latter against turning (put a brake on the LR knob 36). In addition, since the LR brake 102 can make ascent and descent but is incapable of turning, the above-mentioned braking action will by no means cause the LR knob 36 to turn.

Note here that the LR fixing knob 40 is so adapted that by controlling the amount of its turning, the force by which the LR brake 102 depresses the LR knob 36 can be adjusted such that the brake being exerted upon the LR knob 36 takes either a fixed state or a half-braking state.

The fixed state is such that the LR brake 102 depresses the LR knob 36 with such a great force that the latter is incapable of turning. In contrast, the half-braking state is such that the LR knob 36 is sufficiently depressed with the LR brake 102 that it is prevented from automatic turning due, for example, to the counter force of the bent angle portion 24 but that it is actually possible to turn the LR knob 36 although this requires an increased power to turn it on account of the frictional force that has developed.

Preferably, a suitable known means such as sensing a light impact (a feel of click) that results from the advancing end of the LR fixing knob 40 coming into engagement with the recess in the LR knob 36 may be employed to ensure that the operator, when putting a brake on the LR knob 36 by turning the LR fixing knob 40, can learn that the brake is in the half-braking state or in the fixed state.

In addition, the area of contact between the LR brake 102 and the LR knob 36 need be present in the interior of the watertight structure and it is also preferred to design the manipulating section in such a manner that the LR brake 102 is easy to replace.

This remark is equally applicable to the designs, to be described later, of the UD knob 38, UD fixing lever 42, and the like.

As mentioned before, the pulley 96 is fixed to the lower end of the linking tube 94.

The pulley 96 has the wires 60 and 62 fixed thereto as they are connected to the angle portion 24 to bend it to either right or left; the pulley 96 also has a gear formed in such a way that it can be rotated by the LR motor 98.

The LR motor 98 in turn is fixed to the housing 90 of the manipulating section 14 by a stay (not shown) or the like. A gear 104 is fixed to the rotating shaft of the LR motor 98. A gear 106 is axially supported on the housing 90 (by a stay or the like that is fixed thereto, although not shown). The gear 106 meshes with the gear 104 on the rotating shaft of the LR motor 98 and the gear formed on the pulley 96.

Consequently, when the LR motor 98 is driven, the rotating force is transmitted to the pulley 96, assisting in the manipulation of the LR knob 36 that is directly coupled to a pulley 112 via a linking tube 110, namely, assisting in the bending of the angle portion 24 to either right or left.

In the manipulating section 14 of the endoscope 10, the UD knob 38 is provided between the LR knob 36 and the housing 90 (under the LR knob 36).

The UD knob 38 has a recess formed on its underside. The linking tube (rotating shaft) 110 is fixed to the ceiling of the recess and the pulley 112 is fixed to the lower end of the linking tube 110. The pulley 112 has two wires (angle wires) 114 and 116 fixed thereto as they are connected to the angle portion 24 such that they pull it to bend up and down. And like the previously mentioned pulley 96, the pulley 112 also has a gear formed in such a way that it can be rotated by an UD assist motor 118 (which is hereinafter referred to as an UD motor 118) which assists in the bending of the angle portion 24 either up or down.

The linking tube 110 is a cylindrical member through which the linking tube 94 fixed to the LR knob 36 passes and which is inserted into the insertion portion 90a of the housing 90 such that the linking tube 110 is axially supported by the linking tube 94 to be capable of rotation. Since the linking tube 94 passes through the linking tube 110, the pulley 112 at the lower end of the linking tube 110 is located on top of the pulley 96 at the lower end of the linking tube 94.

Hence, both the UD knob 38 and the pulley 112 are so designed that they rotate about the linking tube 94, i.e., they are axially supported by the linking tube 94 to be capable of rotation; when the UD knob 38 is turned by the operator, the pulley 112 is rotated by the same amount so that either of the wires 114 and 116 is pulled and the other wire is advanced.

Note here that as mentioned before, the linking tube 94, hence the LR knob 36 rotates about the central shaft 92. As a result, the linking tube 110, hence the UD knob 38 also rotates about the central shaft 92, whereupon the LR knob 36 and the UD knob 38 which are the manipulative knobs that cause the angle portion 24 to bend will rotate coaxially.

It goes without saying that in order to get the angle portion 24 to be smoothly manipulated for bending, the LR knob 36 and the UD knob 38 are preferably capable of rotation with low friction. To this end, one preferred means is obviously by ensuring that the central shaft 92, linking tube 94 and the linking tube 110 can mutually rotate in a smooth way.

The UD fixing lever 42 is composed of a manipulating lever 42a and a cylindrical portion 42b.

The cylindrical portion 42b is a cylindrical member through which the insertion portion 90a passes in such a way that its upper part is inserted into the recess in the UD knob 38 and this cylindrical portion 42b is axially supported by the insertion portion 90a to be capable of rotation. The manipulating lever 42a is something like the handle of a lever one end of which is fixed to the cylindrical portion 42b and the other end of which projects from the UD knob 38. Thus, by swinging the manipulating lever 42a, the cylindrical portion 42b can be rotated.

Provided on top of the cylindrical portion 42b of the UD fixing lever 42 is a UD brake 120 for locking the UD knob 38 against turning. The UD brake 120 is in a cylindrical form that has the same outside diameter as the cylindrical portion 42b and through which the insertion portion 90a passes to support it in such a manner that like the previously described LR brake 102, it cannot be turned but can make ascent and descent.

When the UD fixing lever 42 (the cylindrical portion 42b) is turned, the UD brake 120 either ascends or descends along the insertion portion 90a by a known means such as a cam mechanism or a screw mechanism.

As mentioned before, the UD brake 120 is supported by the insertion portion 90a in such a manner that it cannot be turned but can make ascent and descent. The UD brake 120 is similar to the previously described LR brake 102 in that in its lowest position, it is fully spaced from the UD knob 38. However, when it ascends, the UD brake 120 does not turn but contacts or depresses the UD knob 38, whereupon frictional force develops to put a brake on the UD knob 38 so that it will not turn.

Like the previously described LR fixing knob 40, the UD fixing lever 42 is so adapted that by controlling the amount of its turning, namely, the amount in which the manipulating lever 42a is manipulated, the force by which the UD brake 120 depresses the UD knob 38 can be adjusted such that the brake being exerted upon the UD knob 38 takes either a fixed state or a half-braking state.

As mentioned before, the pulley 112 is fixed to the lower end of the linking tube 110.

The pulley 112 has the wires 114 and 116 fixed thereto as they are connected to the angle portion 24 to bend it to either up or down; the pulley 112 also has a gear formed in such a way that it can be rotated by the UD motor 118.

The UD motor 118 is fixed to the housing 90 of the manipulating section 14 and a gear 124 is fixed to the rotating shaft of the UD motor 118. A gear 126 is axially supported on the housing 90. The gear 126 meshes with the gear 124 on the rotating shaft of the UD motor 118 and the gear formed on the pulley 112.

Consequently, when the UD motor 118 is driven, the rotating force is transmitted to the pulley 112, assisting in the manipulation of the UD knob 38 that is directly coupled to the pulley 112 via the linking tube 110, namely, the bending of the angle portion 24 either up or down.

As mentioned before, in order to ensure that the manipulative force exerted on each of the manipulative knobs for bending the angle portion 24 is detected accurately, the manipulative force exerted on each manipulative knob has to be sensed in a position somewhere between the motor that assists in bending and the manipulative knob (upstream of the assist motor in the direction in which the manipulative force is transmitted from the manipulative knob toward the assist motor).

To meet this need, the manipulating section shown in FIG. 7 has a torque sensor 130 positioned in the hatched area of the linking tube 94 for sensing the manipulative force (rotational torque) exerted on the LR knob 36. In addition, a torque sensor 132 for sensing the manipulative force exerted on the UD knob 38 is positioned in the hatched area of the linking tube 110.

Thus, the illustrated manipulating section 14 adopts such a preferred embodiment that part of the cylindrical linking tube that is directly coupled to a knob for manipulating the angle portion 24 to bend and which rotates integrally with that knob is designed as a torque sensor (or alternatively, a torque sensor is positioned in that part of the linking tube), whereby the manipulative force exerted on the manipulative knob to bend the angle portion 24 is directly sensed.

As mentioned before, various known types of torque sensors including a torque sensor that uses a strain gage and a magnetostrictive torque sensor may be utilized as the torque sensors to be positioned in the linking tube 94 and the linking tube 110.

As in the previously described case, if the operator turns the LR knob 36 in order to bend the angle portion 24 to either right or left, the applied manipulative force (rotational torque) is sensed by the torque sensor 130 provided in the linking tube 94 directly coupled to the LR knob 36. The sensed manipulative force is processed as in the previously described case and the LR motor 98 is driven in such a way as to assist in the turning of the LR knob 36 by means of a force that accounts for a predetermined proportion of the applied manipulative force or with a force that is equal to the applied manipulative force as multiplied by a predetermined coefficient. The rotational force of the LR motor 98 is transmitted from the gear 104 through the gear 106 to reach the pulley 96 to rotate it. This effectively assists in the manipulation of the LR knob 36, namely, the bending of the angle portion 24 to either right or left, in accordance with the manipulative force exerted on the LR knob 36.

On the other hand, if the operator turns the UD knob 38 in order to bend the angle portion 24 either up or down, the applied manipulative force is similarly sensed by the torque sensor 132 provided in the linking tube 110 directly coupled to the UD knob 38. The sensed manipulative force is processed as in the previously described case and the UD motor 118 is driven in such a way as to assist in the turning of the UD knob 38 by means of a force that typically accounts for a predetermined proportion of the applied manipulative force. When the UD motor 118 is driven, its rotational force is transmitted to the pulley 112 to rotate it, effectively assisting in the bending of the angle portion 24 either up or down in accordance with the manipulative force exerted on the UD knob 38.

In the case shown in FIG. 7, a gear (106 or 126) is provided between an assist motor that assists in the bending of the angle portion 24 and an associated pulley so as to reduce the number of rotations caused by the motor; this is not the sole case of the present invention and the assist motor (motor head) may be provided with a planetary gear wheel or a harmonic drive to reduce the number of rotations caused by the motor; if desired, this mechanism may be combined with a gear to reduce the rotational speed.

In the illustrated case, the rotation of the assist motor is transmitted by gears to rotate the pulley (coupling the inking tube to the manipulative knob) so as to assist in the bending of the angle portion; this is not the sole case of the present invention and a direct drive motor (DD motor) may be used to assist in the bending of the angle portion.

An example of this modification is shown in FIG. 8 by quoting the design depicted in FIG. 7; a cylindrical portion 96a is provided in the lower part of the pulley 96 for bending the angle portion 24 to either right or left; a DD motor with an inner rotor is used as a LR motor 136 whose rotor is in engagement with the cylindrical portion 96a. Rotating the cylindrical portion 96a by means of the LR motor 136 effectively assists in the bending of the angle portion 24 to either right or left.

Similarly, to assist in the bending of the angle portion 24 either up or down, a DD motor with an inner rotor is used as a UD motor 138 whose rotor is in engagement with the linking tube 94 passing through it. Rotating the linking tube 94 by means of the UD motor 138 effectively assists in the bending of the angle portion 24 either up or down.

**Claims**

1. An endoscope comprising:

    an insertion section (12) having a bending portion (24) near a distal end (22) of said insertion section (12);
    manipulating means (36) for manipulating bending of said bending portion (24), wherein manipulating means consists of a left/right knob (36) for bending the bending portion (24) to the left or right and an up/down knob for bending the bending portion (24) up and down
    pulling means (60, 62) for coupling said manipulating means (36) to said bending portion (24) and pulling said bending portion (24) to bend in response to manipulation by said manipulating means (36);
    auxiliary means (72) for assisting in the pulling of said bending portion (24) by said pulling means (60, 62);
    sensing means (78) for sensing a manipulative force applied to said manipulating means (36); and
    control means (76) for controlling the assisting in the pulling of said bending portion (24) by said auxiliary means (72) in accordance with the manipulative force sensed by said sensing means (78),

    **characterized in that** said sensing means (78) is adapted to sense the manipulative force applied to said manipulating means (36) between said auxiliary means (72) and said manipulating means (36).

2. The endoscope according to claim 1, wherein said manipulating means (36) is adapted to be turned to cause said pulling means (60, 62) to pull said bending portion (24), and said sensing means is a torque sensor (78) adapted to sense a torque applied to said manipulating means (36).

3. The endoscope according to claim 1 or 2, wherein said control means (76) is adapted to control drive of said auxiliary means (72) in such a way that in accordance with the manipulative force applied to said manipulating means (36) and sensed by said sensing means (78), it is configured to assist in the pulling of said bending portion (24) by said pulling means (60, 62) with a force that accounts for a predetermined proportion of the applied manipulative force.

4. The endoscope according to any one of claims 1 to 3, wherein said control means (76) is adapted to control drive of said auxiliary means (72) in such a way that, if the manipulative force applied to said manipulating means (36) and sensed by said sensing means (78) is equal to or less than a predetermined value, the pulling of said bending portion by said pulling means (60, 62) is not assisted.

5. The endoscope according to any one of claims 1 to 4, wherein said control means (76) is adapted to control drive of said auxiliary means (72) in such a way that, if the manipulative force applied to said manipulating means (36) and sensed by said sensing means (78) is equal to or less than a predetermined value, the pulling of said bending portion (24) by said pulling means (60, 62) is associated with an auxiliary force that accounts for a smaller proportion of the applied manipulative force than in a case where the applied manipulative force exceeds the predetermined value.

6. The endoscope according to any one of claims 1 to 5, wherein said control means (76) is adapted to control drive of said auxiliary means (72) in such a way that, if the manipulative force applied to said manipulating means (36) and sensed by said sensing means (78) exceeds a predetermined value, the pulling of said bending portion by said pulling means (60, 62) is not assisted.

7. The endoscope according to any one of claims 1 to 6, wherein said control means (76) is adapted to control drive of said auxiliary means (72) in such a way that, if the manipulative force applied to said manipulating means (36) and sensed by said sensing means (78) exceeds a predetermined value, the pulling of said bending portion (24) by said pulling means (60, 62) is assisted with an auxiliary force same as what is produced in a case where the applied manipulative force is of the predetermined value.

8. The endoscope according to any of claims 1 to 7, further comprising:

    a rotating shaft (64) adapted to rotate integrally with said manipulating means (36) to transmit the rotation of said manipulating means (36) to said pulling means (60, 62),

wherein said auxiliary means (72) is adapted to engage directly or indirectly with said rotating shaft (64) to assist in the pulling of said bending portion (24) by said pulling means (60, 62) and said sensing means (78) is adapted to sense the manipulative force applied to said manipulating means (36), by sensing a rotational force on said rotating shaft (64) in a position that is closer to said manipulating means (36) than to a position of engagement of said auxiliary means (72).

**Patentansprüche**

1. Endoskop, umfassend:

   einen Einführabschnitt (12) mit einem Biegeabschnitt (24) nahe einem distalen Ende (220) des Einführabschnitts (12);
   eine Handhabungseinrichtung (36) zum Manipulieren des Biegens des Biegeabschnitts (24), wobei die Handhabungseinrichtung aus einem Links-/Rechts-Knopf (36) zum Biegen des Biegeabschnitts (24) nach links oder rechts und einem Auf-/Ab-Knopf zum Biegen des Biegeabschnitts (24) nach oben und nach unten besteht,
   eine Zugeinrichtung (60, 62) zum Koppeln der Handhabungseinrichtung (36) mit dem Biegeabschnitt (24) und zum Ziehen des Biegeabschnitts (24) zum Biegen ansprechend auf eine Manipulation der Handhabungseinrichtung (36);
   eine Hilfseinrichtung (72) zum Unterstützen beim Ziehen des Biegeabschnitts (24) durch die Zugeinrichtung (60, 62);
   eine Sensoreinrichtung (78) zum Fühlen einer Handhabungskraft, die auf die Handhabungseinrichtung (36) ausgeübt wird; und
   eine Steuereinrichtung (76), zum Steuern der Unterstützung beim Ziehen des Biegeabschnitts (24) durch die Hilfseinrichtung (72) nach Maßgabe der von der Sensoreinrichtung (78) gefühlten Handhabungskraft,
   **dadurch gekennzeichnet, dass** die Sensoreinrichtung (78) dazu ausgebildet ist, die auf die Handhabungseinrichtung aufgebrachte Handhabungskraft zwischen der Hilfseinrichtung (72) und der Handhabungseinrichtung (36) zu fühlen.

2. Endoskop nach Anspruch 1, bei dem die Handhabungseinrichtung (36) dazu ausgebildet ist, gedreht zu werden, um die Zugeinrichtung (60, 62) zu veranlassen, den Biegeabschnitt (24) zu ziehen, und die Sensoreinrichtung ein Drehmomentsensor (78) ist, ausgebildet zum Fühlen eines auf die Handhabungseinrichtung (36) aufgebrachten Drehmoments.

3. Endoskop nach Anspruch 1 oder 2, bei dem die Steuereinrichtung (76) dazu ausgebildet ist, das Antreiben der Hilfseinrichtung (72) derart zu steuern, dass nach Maßgabe der auf die Handhabungseinrichtung (36) aufgebrachten und von der Sensoreinrichtung (78) gefühlten Handhabungskraft sie konfiguriert ist zum Unterstützen beim Ziehen des Biegeabschnitts (24) durch die Zugeinrichtung (60, 62) mit einer Kraft, die einen vorbestimmten Anteil der aufgebrachten Handhabungskraft ausmacht.

4. Endoskop nach einem der Ansprüche 1 bis 3, bei dem die Steuereinrichtung (76) dazu ausgebildet ist, den Antrieb der Hilfseinrichtung (72) derart zu steuern, dass, wenn die auf die Handhabungseinrichtung (36) aufgebrachte und von der Sensoreinrichtung (78) gefühlte Handhabungskraft gleich oder kleiner als ein vorbestimmter Wert ist, das Ziehen des Biegeabschnitts durch die Zugeinrichtung (60, 62) nicht unterstützt wird.

5. Endoskop nach einem der Ansprüche 1 bis 4, bei dem die Steuereinrichtung (76) dazu ausgebildet ist, den Antrieb der Hilfseinrichtung (72) derart zu steuern, dass, wenn die auf die Handhabungseinrichtung (36) aufgebrachte und von der Sensoreinrichtung (78) gefühlte Handhabungskraft gleich oder kleiner als ein vorbestimmter Wert ist, das Ziehen des Biegeabschnitts (24) durch die Zugeinrichtung (60, 62) mit einer Hilfskraft unterstützt wird, die einen kleineren Anteil der aufgebrachten Handhabungskraft ausmacht als in dem Fall, in welchem die aufgebrachte Handhabungskraft den vorbestimmten Wert übersteigt.

6. Endoskop nach einem der Ansprüche 1 bis 5, bei dem die Steuereinrichtung (76) dazu ausgebildet ist, den Antrieb der Hilfseinrichtung (72) derart zu steuern, dass, wenn die auf die Handhabungseinrichtung (36) aufgebrachte und von der Sensoreinrichtung (78) gefühlte Handhabungskraft einen vorbestimmten Wert übersteigt, das Ziehen des Biegeabschnitts durch die Zugeinrichtung (60, 62) nicht unterstützt wird.

7. Endoskop nach einem der Ansprüche 1 bis 6, bei dem die Steuereinrichtung (76) dazu ausgebildet ist, den Antrieb der Hilfseinrichtung (72) in der Weise zu steuern, dass, wenn die von der Handhabungseinrichtung (36) aufgebrachte und von der Sensoreinrichtung (78) gefühlte Handhabungskraft einen vorbestimmten Wert übersteigt, das Ziehen des Biegeabschnitts (24) durch die Zugeinrichtung (60, 62) mit einer Hilfskraft unterstützt wird, welche die gleiche ist wie die, die in dem Fall erzeugt wird, in welchem die aufgebrachte Handhabungskraft dem vorbestimmten Wert entspricht.

8.  Endoskop nach einem der Ansprüche 1 bis 7, weiterhin umfassend:

    eine Drehwelle (64), ausgebildet zum vereinten Drehen mit der Handhabungseinrichtung (36), um die Drehung der Handhabungseinrichtung (36) auf die Zugeinrichtung (60, 62) zu übertragen,
    wobei die Hilfseinrichtung (72) dazu ausgebildet ist, direkt oder indirekt mit der Drehwelle (64) in Eingriff zu treten, um das Ziehen des Biegeabschnitts (24) durch die Zugeinrichtung (60, 62) zu unterstützen, und die Sensoreinrichtung (78) dazu ausgebildet ist, die auf die Handhabungseinrichtung (36) aufgebrachte Handhabungskraft zu fühlen, indem eine Drehkraft an der Drehwelle (64) an einer Stelle gefühlt wird, die näher bei der Handhabungseinrichtung (36) liegt als eine Eingriffsstelle der Hilfseinrichtung (72).


**Revendications**

1.  Endoscope comportant :

    une section d'insertion (12) ayant une partie de flexion (24) près d'une extrémité distale (22) de ladite section d'insertion (12) ;
    des moyens de manipulation (36) destinés à manipuler en flexion ladite partie de flexion (24), les moyens de manipulation se composant d'un bouton gauche/droit (36) destiné à faire fléchir la partie de flexion (24) vers la gauche ou la droite et un bouton haut/bas destiné à faire fléchir la partie de flexion (24) vers le haut et vers le bas
    des moyens de traction (60, 62) destinés à relier lesdits moyens de manipulation (36) à ladite partie de flexion (24) et à tirer ladite partie de flexion (24) afin de fléchir en réponse à la manipulation par lesdits moyens de manipulation (36) ;
    des moyens auxiliaires (72) destinés à aider à la traction de ladite partie de flexion (24) par lesdits moyens de traction (60, 62) ;
    des moyens de détection (78) destinés à détecter une force de manipulation appliquée sur lesdits moyens de manipulation (36) ; et
    des moyens de commande (76) destinés à commander l'assistance à la traction de ladite partie de flexion (24) par lesdits moyens auxiliaires (72) en fonction de la force de manipulation détectée par lesdits moyens de détection (78),
    **caractérisé en ce que** lesdits moyens de détection (78) sont prévus pour détecter la force de manipulation appliquée sur lesdits moyens de manipulation (36) entre lesdits moyens auxiliaires (72) et lesdits moyens de manipulation (36).

2.  Endoscope selon la revendication 1, dans lequel lesdits moyens de manipulation (36) sont prévus pour être tournés afin d'amener lesdits moyens de traction (60, 62) à tirer ladite partie de flexion (24), et lesdits moyens de détection sont constitués par un capteur de couple (78) prévu pour détecter un couple appliqué sur lesdits moyens de manipulation (36).

3.  Endoscope selon la revendication 1 ou 2, dans lequel lesdits moyens de commande (76) sont prévus pour commander l'entraînement desdits moyens auxiliaires (72) d'une manière telle que, en fonction de la force de manipulation appliquée sur lesdits moyens de manipulation (36) et détectée par lesdits moyens de détection (78), ils sont configurés pour assister la traction de ladite partie de flexion (24) par lesdits moyens de traction (60, 62) avec une force qui représente une proportion prédéterminée de la force de manipulation appliquée.

4.  Endoscope selon l'une quelconque des revendications 1 à 3, dans lequel lesdits moyens de commande (76) sont prévus pour commander l'entraînement desdits moyens auxiliaires (72) d'une manière telle que, si la force de manipulation appliquée sur lesdits moyens de manipulation (36) et détectée par lesdits moyens de détection (78) est égale ou inférieure à une valeur prédéterminée, la traction de ladite partie de flexion par lesdits moyens de traction (60, 62) n'est pas assistée.

5.  Endoscope selon l'une quelconque des revendications 1 à 4, dans lequel lesdits moyens de commande (76) sont prévus pour commander l'entraînement desdits moyens auxiliaires (72) d'une manière telle que, si la force de manipulation appliquée sur lesdits moyens de manipulation (36) et détectée par lesdits moyens de détection (78) est égale ou inférieure à une valeur prédéterminée, la traction de ladite partie de flexion (24) par lesdits moyens de traction (60, 62) est associé à une force auxiliaire qui représente une proportion de la force de manipulation appliquée plus petite que dans un cas où la force de manipulation appliquée dépasse la valeur prédéterminée.

6.  Endoscope selon l'une quelconque des revendications 1 à 5, dans lequel lesdits moyens de commande (76) sont prévus pour commander l'entraînement desdits moyens auxiliaires (72) d'une manière telle que, si la force de manipulation appliquée sur lesdits moyens de manipulation (36) et détectée par lesdits moyens de détection (78) dépasse une valeur prédéterminée, la traction de ladite partie de flexion par lesdits moyens de traction (60, 62) n'est pas assistée.

**7.** Endoscope selon l'une quelconque des revendications 1 à 6, dans lequel lesdits moyens de commande (76) sont prévus pour commander l'entraînement desdits moyens auxiliaires (72) d'une manière telle que, si la force de manipulation appliquée sur lesdits moyens de manipulation (36) et détectée par lesdits moyens de détection (78) dépasse une valeur prédéterminée, la traction de ladite partie de flexion (24) par lesdits moyens de traction (60, 62) est assistée avec une force auxiliaire qui est la même que ce qui est produit dans un cas où la force de manipulation appliquée est de la valeur prédéterminée.

**8.** Endoscope selon l'une quelconque des revendications 1 à 7, comportant en outre :

un arbre rotatif (64) prévu pour tourner d'un seul tenant avec lesdits moyens de manipulation (36) afin de transmettre la rotation desdits moyens de manipulation (36) aux dits moyens de traction (60, 62),
lesdits moyens auxiliaires (72) étant prévus pour engager directement ou indirectement ledit arbre rotatif (64) afin d'aider à la traction de ladite partie de flexion (24) par lesdits moyens de traction (60, 62) et lesdits moyens de détection (78) étant prévus pour détecter la force de manipulation appliquée sur lesdits moyens de manipulation (36), en détectant une force de rotation sur ledit arbre rotatif (64) dans une position qui est plus proche desdits moyens de manipulation (36) que dans une position d'engagement desdits moyens auxiliaires (72).

# FIG.1

FIG.2

FIG.3

# FIG.4

## FIG.5A

MANIPULATIVE FORCE F

COEFFICIENT K → AMP → MOTOR 72 → AUXILIARY FORCE FROM MOTOR

CURRENT FEEDBACK

+ + BENDING LOAD W

## FIG.5B

MANIPULATIVE FORCE F

G → COEFFICIENT K → AMP → MOTOR 72 → AUXILIARY FORCE FROM MOTOR

PHASE-DELAY COMPENSATION CIRCUIT

CURRENT FEEDBACK

+ + BENDING LOAD W

EP 2 039 284 B1

# FIG.6A

AUXILIARY FORCE

MANIPULATIVE FORCE

# FIG.6D

AUXILIARY FORCE

MANIPULATIVE FORCE

# FIG.6B

AUXILIARY FORCE    INSENSITIVE ZONE

MANIPULATIVE FORCE

# FIG.6E

AUXILIARY FORCE

MANIPULATIVE FORCE

# FIG.6C

LOW-SENSITIVITY RANGE

AUXILIARY FORCE

MANIPULATIVE FORCE

22

# FIG.7

# FIG.8

**EP 2 039 284 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 3017769 B
- JP 3030129 B
- JP 2005028018 A
- US 5469840 A